# EUROPÄISCHE PATENTSCHRIFT

(11) **EP 1 137 625 B1**
(45) Veröffentlichungstag und Bekanntmachung des Hinweises auf die Patenterteilung: **01.10.2003**
(21) Anmeldenummer: 99963393.6
(22) Anmeldetag: 06.12.1999
(51) Int. Cl.: C07C 209/68, C07C 209/84, C07C 211/63, C25B 3/00

(54) **VERFAHREN ZUR HERSTELLUNG ODER REINIGUNG VON ONIUMHYDROXIDEN MITTELS ELEKTRODIALYSE**
METHODS FOR PRODUCING OR PURIFYING ONIUM HYDROXIDES BY MEANS OF ELECTRODIALYSIS
PROCEDES POUR LA PRODUCTION OU LA PURIFICATION D'HYDROXYDES D'ONIUM PAR ELECTRODIALYSE

(30) Priorität: 07.12.1998 DE 19856376
(43) Veröffentlichungstag der Anmeldung: 04.10.2001
(73) Patentinhaber: BASF AKTIENGESELLSCHAFT, 67056 Ludwigshafen (DE)
(72) Erfinder: BOTZEM, Jörg, D-67117 Limburgerhof (DE); KOBER, Reiner, D-67136 Fu gönheim (DE); FREDE, Markus, D-69214 Eppelheim (DE)
(74) Vertreter: Isenbruck, Günter, Dr.
(86) Internationale Anmeldenummer: EP9909515
(87) Internationale Veröffentlichungsnummer: WO00034224

(56) Entgegenhaltungen:
- EP-A- 0 834 345
- EP-A- 0 834 346
- EP-A- 0 870 532
- WO-A-88/07975
- WO-A-98/09002
- WO-A-98/44169
- US-A- 5 389 211

## Beschreibung

Gegenstand der Erfindung sind Verfahren zur Herstellung oder Reinigung von Oniumhydroxiden der Elemente N, S oder P durch Elektrodialyse in einer Elektrodialysevorrichtung mit einer oder mehreren Zelleinheiten, wobei jede Zelleinheit aus einer bipolaren Membran und einer anionenselektiven Membran besteht.

Stabile oder wenigstens weitgehend stabile Oniumverbindungen, d.h. die Oniumverbindungen der Elemente N, S und P, spielen in vielen Bereichen der chemischen Synthese oder Analyse eine große Rolle. Insbesondere die Oniumverbindungen des Stickstoffs, d.h. die quaternären Ammoniumverbindun-gen, decken ein breites Anwendungsspektrum ab. Die quaternären Ammoniumhydroxide, z.B. Tetramethylammoniumhydroxid (TMAH) und Tetraethylammoniumhydroxid (TEAH) sind starke organische Basen, die bereits seit vielen Jahren bekannt sind. Solche quaternären Ammoniumhydroxide haben eine große Zahl von Anwendungen gefunden, beispielsweise zur Titration von Säuren in organischen Lösemitteln oder als Elektrolyte in der Polarographie. Wäßrige Lösungen von quaternären Ammoniumhydroxiden, insbesondere von TMAH werden häufig als Entwickler für Photoresists in gedruckten Schaltungen und bei der Chipherstellung eingesetzt. Oft erfordert die jeweilige Anwendung jedoch, daß neben den oben bereits genannten technisch in großen Mengen verfügbaren Ammoniumhydroxiden solche mit größeren organischen Substituenten eingesetzt werden, beispielsweise bei der Anwendung als Phasentransferkatalysatoren oder bei der Zeolithherstellung.

Bei vielen Anwendungen ist es zudem erforderlich, daß die Ammoniumhydroxide eine hohe Reinheit ausweisen, um beispielsweise die Bildung von Nebenprodukten oder die Verunreinigung von Halbleiterelementen zu verhindern. Die geforderte hohe Reinheit bezieht sich auf Restmengen an Halogeniden, Sulfaten, Carbonaten und dergleichen, beispielsweise bei der Herstellung von Halbleiterelementen. Wenn Ammoniumhydroxide bei der Zeolithherstellung eingesetzt werden, so bezieht sich die hohe Reinheit insbesondere auf einen möglichst niedrigen Gehalt an Alkalimetallionen.

Verschiedene Verfahren zur Herstellung von quaternären Ammoniumhydroxiden, wie TMAH, TEAH oder TPAH, sind bereits bekannt. Im allgemeinen werden die quaternären Ammoniumhydroxide durch Elektrolyse eines Salzes einer quaternären Ammoniumverbindung in einer elektrochemischen Zelle hergestellt, die eine oder mehrere Membranen aufweist, die zum Austausch von Kationen fähig sind. Die üblicherweise bei solchen Verfahren eingesetzten quaternären Ammoniumsalze umfassen Halogenide, Carboxylate, Carbonate und Sulfate.

Die WO 98/09002 beschreibt die Herstellung von Oniumhydroxiden in einer elektrochemischen Zelle. Die Druckschrift beschreibt ein Elektrodialyseverfahren unter Verwendung einer Zelleinheit, die vier Volumina aufweist, wobei die Zelleinheit durch eine anodenseitige bipolare Membran, eine erste, die Zelle teilende Membran und eine zweite, die Zelle teilende Membran definiert ist. Detailliert beschrieben werden Zellaufbauten, die unterschiedliche Anordnungen bipolarer Membranen mit jeweils einer anionenselektiven und einer oder mehreren kationenselektiven Membranen als Zellteiler aufweisen.

Die EP 0 834 346 beschreibt ein Verfahren zur Reinigung von Hydroxyverbindungen in einer elektrochemischen Zelle, wobei die Zelleinheit eine bipolare Membran und eine kationische Membran aufweist. Beschrieben werden weiterhin Zellanordnungen, die neben der auf zwei Membranen aufgebauten Zelle noch weitere Membranen, in der Regel anionische Membranen, aufweisen.

Die WO 98/44169 betrifft ein Verfahren zur Herstellung von Oniumhydroxiden aus einem entsprechendem Oniumsalz und zur Reinigung solcher Oniumhydroxide. Beschrieben wird u.a. eine Anordnung elektrochemischer Zellen, wobei mindestens eine erste elektrochemische Zelle und eine zweite elektrochemische Zelle eingesetzt weiden. Die erste Art elektrochemischer Zellen weist eine bipolare Membran und mindestens eine weitere Zellbegrenzung auf, wobei die mindestens eine weitere Zellbegrenzung beispielsweise eine nichtionische mikroporöse Diffusionsbarriere wie Sieb, Filter, Diaphragma etc. oder eine ionische Zellbegrenzung, wie eine kationenselektive Membran sein kann. Die zweite elektrochemische Zelle kann eine oder mehrere bipolare Membranen und einen oder mehrere Zellteiler aufweisen, wobei als Zellteiler kationenselektive. Membranen und anionenselektive Membranen als mitumfaßt angegeben werden. Gemäß der WO 98/44169 dient die zweite Zellanordnung dem Zweck, unerwünschte Mengen an Säure aus der Lösung zu entfernen, die ursprünglich in den Kreislauf eingespeist wurde. Der Einsatz einer Elektrodialyseeinheit, bei der eine Einheitszelle eine bipolare und eine anionische Membran aufweist, wobei sich anodenseitig zwischen der letzten anionenselektiven Membran und der Anode eine bipolare Membran oder eine kationenselektive Membran befindet, zur Herstellung von Oniumhydroxiden wird in der Druckschrift nicht offenbart J.R. Ochoa, Gomez und M. Tarancon Estrada beschreiben in *Journal ofApplied Electrochemistry*, **21,** (1991), Short Communication, die Synthese quaternärer Ammoniumhydroxide in einer Elektrolysezelle, die eine Anionenaustauschmembran aufweist. Gemäß dem in der Druckschrift beschriebenen Verfahren werden quaternäre Ammoniumhydroxide erhalten, die noch einen hohen Gehalt an Halogeniden aufweisen.

Die US-A 4 578 161 beschreibt ein Verfahren zur Herstellung von quaternären Ammoniumhydroxiden durch Elektrolyse, wobei eine Elektrolysezelle eingesetzt wird, die eine Anionenaustauschmembran aufweist. Die nach dem beschriebe-nen Verfahren erhaltenen Lösungen von quaternären Ammoniumhydroxiden zeichnet sich ebenfalls durch einen hohen Restgehalt an Bromid aus.

Die US-A 5 286 354 betrifft ebenfalls ein Verfahren zur Herstellung von anorganischen Hydroxiden und Alkoxiden durch Elektrolyse. Das beschriebene Verfahren betrifft den Einsatz einer Elektrolysezelle, bei der die Volumina welche die jeweilige Elektrode enthalten durch eine Anionenaustauschmembran getrennt sind. Die nach dem beschrieben Verfahren erhaltenen Lösungen anorganischer Hydroxide weisen sehr hohe Restgehalte an Bromid auf.

Da elektrodialytische Verfahren in der Regel mit mehr als nur einer Zelleinheit durchgeführt werden, führt ein komplexer Aufbau einer Zelleinheit, wie er im Stand der Technik oft beschrieben wird, in Abhängigkeit von der Zahl der in der Elektrodialysevorrichtung verwendeten Zelleinheiten zu einem kostspieligen und störanfälligen System. Es ist daher wünschenswert, die Komplexität der Zelleinheit so gering wie möglich zu halten, um die Kosten der Elelctrodialysevorrichtung gering und die Stabilität so hoch wie möglich zu halten. Ein Nachteil der aus dem Stand der Technik bekannten Systeme und Elektrodialysevorrichtungen besteht weiterhin darin, daß die voluminösen Oniumionen, beispielsweise quaternäre Ammoniumionen, während des Elektrodialysevorgangs eine kationenselektive Membran passieren müssen. Durch die limitierte Porengröße einer solchen Membran ist es mit dem aus dem Stand der Technik bekannten Verfahren nicht möglich, Oniumhydroxide herzustellen, deren Kationen ein großes Volumen anweisen. Die Herstellung solcher Oniumhydroxide erforderte bislang daher einen komplexen Zellaufbau, der die Sicherheit der Etektrodialysevorrichtung beeinträchtigt und die Wirtschaftlichkeit eines solchen Herstellungsverfahrens beeinträchtigt.

Zudem weisen die aus dem Stand der Technik bekannten Zellaufbauten einen weiteren Nachteil auf, da sich produkthaltige Stoffströme in direktem Kontakt mit der Anode bzw. mit der Kathode befinden. Dieser Kontakt kann zur Bildung von Verunreinigungen in den Stoffströmen sowie zu einer verminderten Lebensdauer des Anoden- bzw. Kathodenmaterials führen.

Es war daher eine Aufgabe der vorliegenden Erfindung, ein Verfahren zur Herstellung von Oniumhydroxiden bzw. ein Verfahren zu deren Reinigung zur Verfugung zu stellen, das einen einfachen Aufbau einer Zelleinheit gewährleistet, die Herstellung von Oniumhydroxiden ermöglicht, die Kationen großen Volumens aufweisen und zudem die Lebensdauer des Anoden- und Kathodenmaterials verlängert, wobei gleichzeitig Stoffströme mit einem geringen Gehalt an Verunreinigungen erhalten werden.

Die erfindungsgemäße Lösung dieser Aufgabe wird durch ein Verfahren bereitgestellt, bei dem ein Salz einer Oniumverbindung in einer Elektrodialysevorrichtung mittels eines Elektrodialyseverfahrens in das Oniumhydroxid überführt wird, wobei eine Zelleinheit der Elektrodialysevorrichtung aus einer bipolaren Membran und einer anionenselektiven Membran besteht, wobei sich anodenseitig zwischen der letzten anionenselektiven Membran und der Anode eine bipolare Membran oder eine kationenselektive Membran befindet.

Gegenstand der Erfindung ist daher ein Verfahren zur Herstellung von Oniumhydroxiden der Elemente N, S oder P durch Elektrodialyse in einer Elektrodialysevorrichtung, die eine Anode, eine Kathode sowie eine oder mehrere Zelleinheiten mit jeweils einem Säure- und Basekreislauf aufweist, bei dem eine Lösung eines Oniumsalzes der allgemeinen Formel (I) 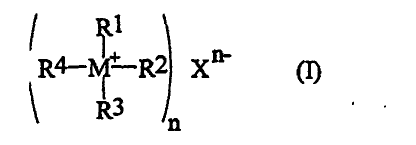 worin M für N, S oder P; R¹, R², R³ und R⁴ jeweils unabhängig voneinander für einen linearen oder verzweigten, gesättigten oder ungesättigten aliphatischen, cycloaliphatischen, araliphatischen oder aromatischen Rest mit 1 bis 20 C-Atomen stehen oder zwei der Reste R¹ bis R⁴ zusammen mit M einen heterocyclischen Ring bilden, X⁻ für ein n-wertiges Anion und n für eine Zahl von 1 bis 4 steht, in den Basekreislauf einer Zelleinheit eingebracht und einer Elektrodialyse unterzogen wird, dadurch gekennzeichnet, daß jede Zelleinheit aus einer bipolaren Membran und einer anionenselektiven Membran besteht und wobei sich anodenseitig zwischen der letzten anionenselektiven Membran und der Anode eine bipolare Membran oder eine kationenselektive Membran befindet.

In einer bevorzugten Ausführungsform der vorliegenden Erfindung steht M für Stickstoff (N).

Die Reste R¹, R², R³ und R⁴ können jeweils unabhängig voneinander für einen linearen oder verzweigten, gesättigten oder ungesättigten aliphatischen, cycloaliphatischen, araliphatischen oder aromatischen Rest mit etwa 1 bis etwa 20 C-Atomen stehen. Beispiele für solche Reste sind Methyl-, Ethyl-, Propyl-, Isopropyl, n-Butyl-, Isobutyl, tert-Butyl, Pentyl-, Hexyl-, Heptyl-, Octyl-, Isooctyl-, Nonyl-, Decyl-, Isodecyl-, Dodecyl-, Tridecyl-, Isotridecyl-, Hexadecylund Oktadecylgruppen. R¹, R², R³ und R⁴ können zudem gegebenenfalls mit funktionellen Gruppen substituiert sein. Beispiele für solche fünktionellen Gruppen sind Hydroxygruppen oder Estergruppen.

Beispiele für mit Hydroxygruppen substituierte Reste R¹ bis R⁴ sind Hydroxyethyl, Hydroxypropyl, Hydroxybutyl, Hydroxypentyl und deren höhere Homologen sowie Isomere davon.

In einer bevorzugten Ausführungsform der vorliegenden Erfindung stehen R¹ bis R⁴ jeweils unabhängig voneinander für Alkylgruppen mit 1 bis 10 C-Atomen und insbesondere lineare oder verzweigte Alkylreste mit etwa 2 bis etwa 5 C-Atomen. In einer bevorzugten Ausführungsform der Erfindung stehen R¹ bis R⁴ jeweils unabhängig voneinander für Propyl, Isopropyl oder Butyl.

Die Reste R¹ bis R⁴, wie sie oben definiert wurden, können weiterhin Alkoxysubstituenten aufweisen. Beispiele für solche Reste R¹ bis R⁴ sind Ethoxyethyl, Butoxymethyl, Butoxybutyl und dergleichen.

X steht in Formel (I) für ein n-wertiges Anion. Vorzugsweise handelt es sich dabei um ein Anion einer Brönsted-Säure. In einer bevorzugten Ausführungsform der Erfindung steht X für F, Cl, Br, I, SO₄, R⁵SO₄, HSO₄, CO₃, HCO₃, R⁵CO₃ oder R⁵CO₂ oder ein Gemisch aus zwei oder mehr davon, wobei R⁵ für einen linearen oder verzweigten, gesättigten oder ungesättigten aliphatischen, cycloaliphatischen, araliphatischen oder aromatischen Rest mit 1 bis 20 C-Atomen steht.

In einer weiteren bevorzugten Ausführungsform steht X für ein Anion aus der Gruppe der Halogene, insbesondere für Cl und Br.

Beispiele für Oniumsalze der Formel (I), die im Rahmen der vorliegenden Erfindung eingesetzt werden können, sind Tetramethylammoniumchlorid, Tetramethylammoniumbromid, Tetraethylammoniumchlorid, Tehaethylammoniumbromid, Tetrapropylammoniumchlorid, Tetrapropylammoniumbromid, Tetra-n-octylammoniumbromid, Tetraisopropylammoniumchlorid, Tetraisopropylammoniumbromid, Trimethylhydroxyethylammoniumchlorid, Trimethylmethoxyethylammoniumchlorid, Tripropylhydroxyethylammoniumchlorid, Tripropylmemoxyemylammoniumchlorid, Triisopropylhydroxyethylammoniumchlorid, Triisopropylmethoxyethylammoniumchlorid, Dimethyldihyroxyethylammoniumchlorid, Methyltrihydroxyethylammoniumchlorid, Phenyltrimethylammoniumchlorid, Phenyltriethylammoniumchlorid, Benzyltrimethylammoniumchlorid, Benzyltriethylammoniumchlorid, Dimethylpyrrolidoniumbromid, Dimethylpiperidinium-bromid, Diisopropylimidazoliniumbromid, N-Alkylpyridiniumbromide und dergleichen. Die entsprechenden quatemären Ammoniumsulfate, Carbonate, Phosphate und Carboxylate können ebenso eingesetzt werden.

Beispiele für quaternäre Phosphoniumhalogenide, wie sie durch Formel (I) beschrieben werden und im Rahmen der vorliegenden Erfindung eingesetzt werden können, sind Tetramethylphosphoniumbromid, Tetraethylphosphoniumbromid, Tetrapropylphosphoniumbromid, Tetrabutylphosphoniumbromid, Trimethylhydroxyethylphosphoniumbromid, Dimethyldihydroxyethylphosphoniumbromid, Methyltrihydroxyethylphosphoniumbromid, Phenyltrimethylphosphoniumbromid, Phenyltriethylphosphoniumbromid oder Benzyltrimethylphosphoniumbromid. Ebenso einsetzbar sind die oben genannten Verbindungen, bei denen als Anion eines der bereits im Rahmen der Beschreibung der Formel (I) genannten Anionen vorliegt.

In einer bevorzugten Ausführungsform der vorliegenden Erfindung wird als Oniumsalz Tetrapropylammoniumbromid eingesetzt.

Das erfindungsgemäße Verfahren wird in Elektrodialysevorrichtungen mit einer Vielzahl dünner Membranen durchgeführt. Elektrodialysevorrichtungen weisen üblicherweise einen stapelartigen Aufbau auf. Ein solcher Stapel besteht in der Regel aus Elektroden (Anoden und Kathode) an beiden Enden des Stapels und einer Reihe von Membranen und Dichtungen, die eine Vielzahl von separaten Volumina bilden, die durch die Membranen voneinander getrennt sind. Häufig werden die Volumina, welche die Elektroden enthalten, mit Elektrolytflüssigkeiten befüllt, die üblicherweise Anolyt und Katholyt genannt werden.

Der Stapel zwischen den Elektroden weist häufig eine Vielzahl sich wiederholender Membrananordnungen auf. Eine einzelne sich pro Stapel mehrfach wiederholende Membranfolge wird üblicherweise und insbesondere im Rahmen des vorliegenden Textes als Zelleinheit bezeichnet. Eine solche Zelleinheit weist in der Regel mehrere parallele Volumina zwischen den einzelnen, die Zelleinheit bildenden Membranen auf. Eine der Elektrodialyse zu unterziehende Lösung wird in der Regel durch eine der Anzahl der Volumina der Zelleinheiten des gesamten Stapels bestimmte Zahl von Zuleitungen befüllt. Stapel, welche die Gesamtheit der Elektrodialysevorrichtung bilden, können beispielsweise nur eine Art von Zelleinheit aufweisen, es können jedoch auch mehrere verschiedene Arten von Zelleinheiten pro Stapel vorliegen.

Eine Elektrodialysevorrichtung, wie sie im Rahmen der vorliegenden Erfindung zum Einsatz kommt, weist eine oder mehrere Zelleinheiten mit besonders einfachem Aufbau auf. Die Zahl m der pro Elektrodialysevorrichtung verwendeten Zelleinheiten kann bis zu etwa 1000 betragen, in einer bevorzugten Ausführungsform der vorliegenden Erfindung liegt sie zwischen etwa 50 und etwa 150, beispielsweise bei etwa 100.

Eine im Rahmen des erfindungsgemäßen Verfahrens eingesetzte Zelleinheit besteht aus einer bipolaren Membran und einer anionenselektiven Membran.

Bipolare Membranen weisen eine anionenselektive und eine kationenselektive Schicht eines Ionenaustauschmaterials auf. Um Wasser spalten zu können, müssen die Schichten der bipolaren Membran derart ausgerichtet sein, daß die anionenselektive Membranschicht in Richtung der Anode und die kationenselektive Membranschicht in Richtung der Kathode liegt. Wenn durch eine solche Anordnung ein Strom fließt, wird eine zwischen den beiden Membranen der bipolaren Membran liegende Wasserschicht in Hydroxylionen auf der Anodenseite und Protonen auf der Kathodenseite aufgespalten.

Einen weiteren Bestandteil der Zelleinheit stellt eine anionenselektive Membran dar. Die anionenselektive Membran ist zwischen der bipolaren Membran und der Anode, also anodenseitig von der bipolaren Membran, angeordnet.

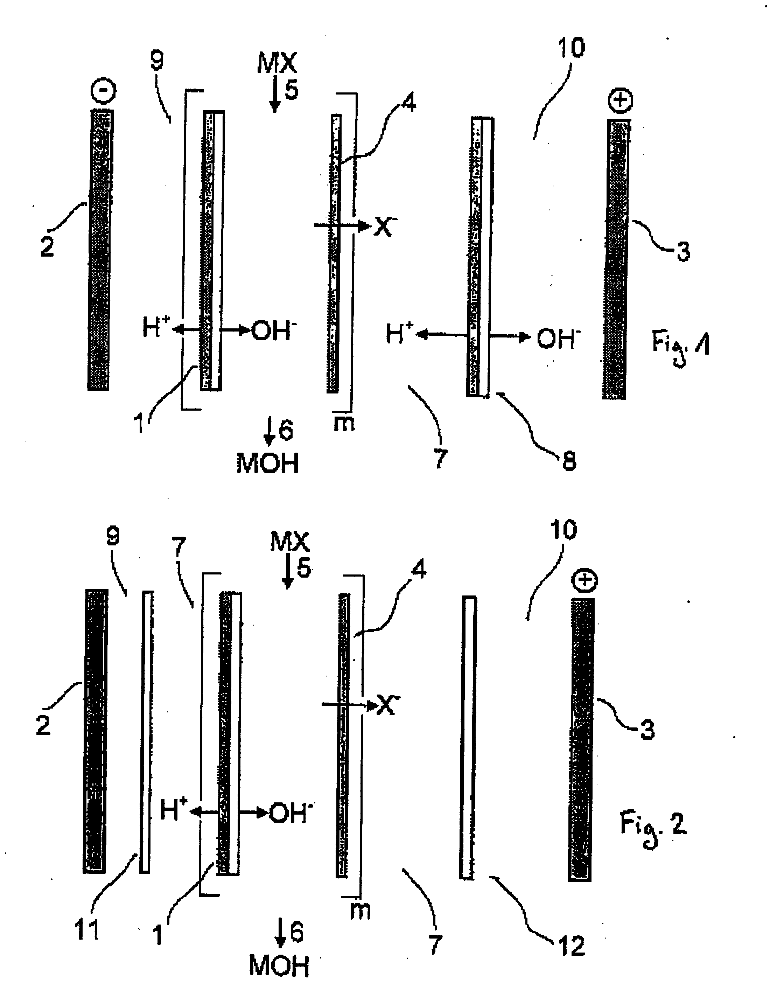

Die in Fig.1 dargestellte Ausführungsform fällt nicht in den Bereich der vorliegenden Erfindung.

Fig. 1 erläutert den Aufbau einer Zelleinheit. Eine bipolare Membran (1) erzeugt im zwischen Kathode (2) und Anode (3) liegenden elektrischen Feld durch Wasserspaltung H⁺ und OH⁻ Ionen. Anodenseitig zur bipolaren Membran ist eine Anionenaustauschermembran (4) angeordnet, die für Anionen X⁻ durchlässig ist. Das Oniumsalz MX (5) wird nun in das zwischen bipolarer Membran und Anionenaustauschermembran befindliche Volumen gefüllt. Während des Elektrodialysevorgangs wandert das Anion X⁻ durch die Anionenaustauschermembran im elektrischen Feld zwischen Kathode (2) und Anode (3) in Richtung Anode. Das Kation M⁺ wird durch die Anionenaustauschermembran zurückgehalten. Mit Fortschreiten der Elektrodialyse finden sich daher im Volumen zwischen bipolarer Membran (1) und Anionenaustauschermembran (4) nur noch OH⁻ Ionen als Gegenionen für M⁺ (6). Das von bipolarer Membran (1) und Anionenaustauschermembran (4) umschlossene Volumen wird im Rahmen des vorliegenden Textes auch "Basekreislauf" genannt.

Anodenseitig zur Anionenaustauschermembran kann das Anion X⁻ mit einem in der bipolaren Membran (1) der darauffolgenden Zelleinheit erzeugten Proton koordinieren.

Wie in Fig. 1 dargestellt, wird zwischen die letzte anodenseitig angeordnete Anionenaustauschermembran (4) und die Anode (3) eine weitere bipolare Membran (8) eingefügt. Dies dient dem Schutz der Anode vor Korrosion und dem Vermeiden chemischer Verunreinigung. Das durch Anionenaustauschermembran (4) und eine darauffolgende bipolare Membran (1 oder 8) gebildete Volumen (7) wird im Rahmen des vorliegenden Textes "Säurekreis" genannt. Die zwischen Kathode (2) und Anode (3) und der jeweils nächsten bipolaren Membran (1 oder 8) liegenden Volumina werden Katholyt (9) und Anolyt (10) genannt. Die Zahl m steht für die Zahl der in Fig. 1 in eckigen Klammern eingeschlossenen Zelleinheiten.

Fig. 2 zeigt eine weitere Zelleinheit, die im Rahmen einer bevorzugten Ausführungsform im erfindungsgemäßen Verfahren eingesetzt wird. Im Unterschied zum in Fig. 1 beschriebenen Zellaufbau fehlt die bipolare Membran (8), und es sind als Begrenzungsmembranen kathodenseitig und anodenseitig jeweils eine Kationenaustauschermembran (11) und (12) vorhanden. Die in Fig. 2 beschriebene Membrananordnung verhindert den direkten Kontakt der Verbindungen in den mit (7) bezeichneten Kreisläufen mit der Anode bzw. der Kathode, wodurch unerwünschte Nebenreaktionen bzw. Kontamination der Elektroden verhindert werden kann.

In einer bevorzugten Ausführungsform der Erfindung ist daher die Kathode der Elektrodialysevorrichtung von der kathodenseitig ersten Zelleinheit durch eine kationenselektive Membran getrennt.

In einer weiteren bevorzugten Ausführungsform der Erfindung ist die Anode der Elektrodialysevorrichtung von der anodenseitig ersten Zelleinheit durch eine kationenselektive Membran und die Kathode der Elektrodialysevorrichtung von der kathodenseitig ersten Zelleinheit ebenfalls durch eine kationenselektive Membran getrennt.

Im Rahmen des erfindungsgemäßen Verfahrens wird nun eine Lösung eines Oniumsalzes MX (5) in den Basekreislauf der in der Elektrodialysevorrichtung enthaltenen Zelleinheiten eingebracht. Die Elektrodialyse wird mit einer Stromdichte von etwa 1 bis etwa 20 A/dm², insbesondere bei etwa 5 bis etwa 10 A/dm² durchgeführt.

Die Temperatur während der Elektrodialyse beträgt etwa 20 bis etwa 60°C, insbesondere etwa 30 bis etwa 50°C, beispielsweise etwa 40°C.

Das erfindungsgemäße Verfahren wird üblicherweise als Batch-Verfahren angewandt.

Hierbei wird die Elektrodialyse für einen Zeitraum von etwa 12 bis 100 Stunden, insbesondere etwa 20 bis etwa 60 Stunden durchgeführt, Leitfähigkeit und Stromstärke können dabei während der gesamten Laufzeit konstant bleiben, es ist jedoch ebenso möglich, das erfindungsgemäße Verfahren mit während der Laufzeit variablen Parametern zu betreiben.

Das Oniumsalz MX wird im Rahmen der vorliegenden Erfindung in der Regel ab dem Basenkreis in Form einer Lösung in einem protischen Lösungsmittel oder einem Gemisch aus zwei oder mehr protischen Lösungsmitteln zugeführt. Als protische Lösungsmittel eignen sich beispielsweise Wasser oder Alkohole, wie Methanol, Ethanol, Propanol, Butanol und dgl. Gegebenenfalls kann als Lösemittel ein Gemisch aus Wasser und einer wasserlöslichen, OH-Gruppen tragenden Verbindung oder einem Gemisch aus zwei oder mehr solcher Verbindungen eingesetzt werden.

Die Konzentration des zugegebenen Oniumsalzes im protischen Lösemittel oder dem Gemisch aus zwei oder mehr protischen Lösemitteln beträgt etwa 0,1 bis etwa 70 Gew.-%, vorzugsweise etwa 1 bis etwa 60 Gew.-%. In einer bevorzugten Ausführungsform der vorliegenden Erfindung beträgt die Konzentration etwa 5 bis 40 Gew.-%, beispielsweise etwa 10, 15 oder 20 Gew.-%.

Anolyt- und Katholytkreislauf können beispielsweise mit Wasser oder dem für das Oniumsalz verwendeten Lösemittel gefüllt sein, es hat sich jedoch als vorteilhaft erwiesen, wenn beide Kreisläufe mit einer Brönsted-Säure, beispielsweise einer Schwefelsäurelösung mit einer Konzentration von etwa 1 bis 10 Gew.-% befüllt werden.

Zur Einstellung der Leitfähigkeit wird in einer bevorzugten Ausführungsform der Erfindung der Säurekreis mit einer etwa 0,1 bis 1 Gew.-%igen Lösung einer Säure HY befüllt, wobei Y ein beliebiges Anion einer Brönsted-Säure sein kann, in einer bevorzugten Ausführungsform der vorliegenden Erfindung jedoch für das Anion X des Oniumsalzes des MX steht.

Als bipolare Membranen eignen sich alle kommerziell erhältlichen Membranen wie BP-1 (Hersteller: Tokuyama Corp.) oder die auf Polysulfonbasis hergestellten Typen von Aqualytics.

Als Anionenaustauschermembranen können ebenfalls alle bekannten Typen eingesetzt werden. In einer bevorzugten Ausführungsform der vorliegenden Erfindung werden die Typen Neosepta AM3, ACLE-SP, AMH oder AHA-2 (Hersteller: Tokuyama Corp.) oder Selemion AMH oder AMP (Hersteller: Asahi Glass) oder Ionac-Membranen der MA-Serie, beispielsweise MA 3148, 3236 oder 3475 (Hersteller: SYBRON), eingesetzt.

Als Kationenaustauschermembranen können alle gängigen Typen eingesetzt werden. Im Rahmen einer bevorzugten Ausführungsform werden die Membranen CMH, CMX, C66-10F (Hersteller: Tokuyama Corp.) oder Membranen der NAFION-Reihe, beispielsweise 117, 350 oder 450 (Hersteller: DuPont) eingesetzt.

Als Anodenmaterialien können eine Vielzahl von Materialien eingesetzt werden. Beispielsweise können Metallanoden, wie titaniumbeschichtete Elektroden, Tantal, Zirkonium, Hafnium oder deren Legierungen eingesetzt werden. Im allgemeinen weisen die Anoden einen nicht passivierbaren und katalytischen Film auf, der Edelmetalle, wie Platin, Iridium, Rhodium oder deren Legierungen oder eine Mischung elektrisch leitfähiger Oxide enthält wobei mindestens eines der Oxide ein Edelmetall wie Platin, Iridium, Ruthenium, Palladium oder Rhodium enthält.

Die Kathoden können ebenfalls jegliches leitfähiges Material aufweisen. Vorzugsweise ist das leitfähige Material unter den herrschenden Bedingungen stabil, und die Kathode weist ein Material auf, das eine geringe Überspannung für Wasserstoffentwicklung aufweist. Beispiele für als Kathoden verwendbare Materialien umfassen rostfreien Stahl, Nickel, Titan, Graphit oder Eisen.

Das im Rahmen des vorliegenden Textes beschriebene, erfindungsgemäße Verfahren kann zur Herstellung von Oniumsalzen der Elemente N, S und P eingesetzt werden. Eine weitere Anwendungsmöglichkeit besteht jedoch darin, beispielsweise Oniumhydroxide der genannten Elemente einer Reinigung zu unterziehen. Hierzu wird der Basekreislauf der Zelleinheiten mit einer entsprechenden, in Wasser dissoziierbare Verunreinigungen enthaltenden Lösung eines Oniwnhydroxids beschickt und der Elektrodialyse unterzogen.

Gegenstand der Erfindung ist demnach auch ein Verfahren zur Reinigung von Oniumhydroxiden der Elemente N, S oder P durch Elektrodialyse in einer Elektrodialysevorrichtung mit einer Anode, einer Kathode sowie einer oder mehreren Zelleinheiten, die jeweils einen Säuren- und einen Basekreislauf aufweisen, bei dem eine in Wasser dissoziierbare Verunreinigungen enthaltende Lösung eines Oniumhydroxids der allgemeinen Formel (I), worin M für N, S oder P; R¹, R², R³ und R⁴ jeweils unabhängig voneinander von einer linearen oder verzweigten, gesättigten oder ungesättigten aliphatischen, cycloaliphatischen, araliphatischen oder aromatischen Rest mit 1 bis 20 C-Atomen, X für OH und n für die Zahl 1 steht, in den Basekreislauf der Zelleinheit eingebracht und einer Elektrodialyse unterzogen wird, dadurch gekennzeichnet, daß jede Zeiteinheit aus einer bipolaren Membran und einer anionenselektiven Membran besteht, wobei sich anodenseitig zwischen der letzten anionenselektiven Membran und der Anode eine bipolare Membran oder eine kationenselektive Membran befindet.

Die Erfindung wird nachfolgend durch weitere Beispiele erläutert:

### BEISPIELE

### Beispiel 1

In eine Dreikreiselektrodialysezelle entsprechend Fig. 2, bestehend aus einer Ruthenium-mischoxidanode, einer Stahlkathode und fünf Zelleinheiten, bipolaren Membranen (Typ Polysulfon, Aqualytics), Anionenaustauschermembranen (ACLE-SP Tokuyama Corp.) und Kationenaustauschermembranen (C66-10F, Tokuyama Corp.) wurde der Basenkreis mit einer 10 Gew.-%igen Tetrapropylammoniumbromid-Lösung befüllt. Der Säurekreis wurde mit einer 0,6 %igen HBr-Lösung beschickt, während im Anolyt und Katholyt-Kreislauf eine 5 %ige Schwefelsäurelösung zirkulierte. Die Temperatur betrug in allen Kreisläufen 40°C. Bei einer Stromstärke von 8 A/dm² und einer Leitfähigkeit von 100 mS im Säurekreis erhielt man eine 8,74-%ige Tetrapropylammoniumhydroxid-Lösung mit einem Restbromid-Gehalt von 27 ppm. Der Restgehalt an Na- und K-Ionen betrug ≤ 1 ppm.

### Beispiel 2

Unter identischen Bedingungen wie in Beispiel 1 erhält man bei 5,7 A/dm² eine 8,50%-ige Tetrapropylammoniumhydroxid-Lösung mit einem Restbromid-Gehalt von 20 ppm. Der Gehalt an Na- und K-Ionen beträgt ≤ 1 ppm.

### Beispiel 3

Unter den identischen Bedingungen wie in Beispiel 1 wurde eine 10 Gew.-%-ige Tetrabutylammoniumbromidlösung elektrodialysiert. Bei einer Stromstärke von 8 A/dm² erhielt man 8,50%-ige Tetrabutylammoniumhydroxid-Lösung mit einem Restbromid-Gehalt von 20 ppm.

## Patentansprüche

1. Verfahren zur Herstellung von Oniumhydroxi den der Elemente N, S oder P durch Elektrodialyse in einer Elektrodialysevorrichtung, die eine Anode (3), eine Kathode (2) sowie eine oder mehrere Zelleinheiten mit jeweils einem Säuren(7)- und einem Basenkreislauf aufweist, bei dem eine Lösung (5) eines Oniumsalzes der allgemeinen Formel I 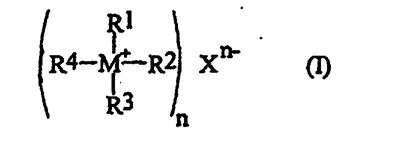 worin M für N, S oder P; R¹, R², R³ und R⁴ jeweils unabhängig voneinander für einen gegebenenfalls mit funktionellen Gruppen substituierten, linearen oder verzweigten, gesättigten oder ungesättigten aliphatischen, cycloaliphatischen, araliphatischen oder aromatischen Rest mit 1 bis 30 C-Atomen stehen oder zwei der Reste R¹ bis R⁴ zusammen mit M einen heterocyclischen Ring bilden, X⁻ für ein n-wertiges Anion und n für eine Zahl von 1 bis 4 steht,
in den Basenkreislauf der Zelleinheit eingebracht und einer Elektrodialyse unterzogen wird, **dadurch gekennzeichnet, daß** jede Zelleinheit aus einer bipolaren Membran (1) und einer anionenselektiven Membran (4) besteht und sich anodenseitig zwischen der letzten anionenselektiven Membran und der Anode (3) eine bipolare Membran (8) oder eine kationenselektive Membran (12) befindet und die Kathode (2) der Elektrodialysevorrichtung von der kathodenseitig ersten Zelleinheit durch eine kationenselektive Membran (11) getrennt ist.

2. Verfahren nach Anspruch 1, **dadurch gekennzeichnet, daß** R¹, R², R³ und R⁴ jeweils unabhängig voneinander für einen linearen oder verzweigten aliphatischen Rest mit 1 bis 4 C-Atomen stehen.

3. Verfahren nach Anspruch 1 oder 2, **dadurch gekennzeichnet, daß** X für das Anion einer Brönsted-Säure steht.

4. Verfahren nach einem der Ansprüche 1 bis 3, **dadurch gekennzeichnet, daß** X für F, Cl, Br, J, SO₄, R⁵SO₄, HSO₄, CO₃, HCO₃, R⁵CO₃, oder R⁵CO₂ oder ein Gemisch aus zwei oder mehr davon und R⁵ für einen linearen oder verzweigten, gesättigten oder ungesättigten aliphatischen, cycloaliphatischen, araliphatischen oder aromatischen Rest mit 1 bis 30 C-Atomen steht.

5. Verfahren nach einem der Ansprüche 1 bis 4, **dadurch gekennzeichnet, daß** M für N steht.

6. Verfahren nach einem der Ansprüche 1 bis 5, **dadurch gekennzeichnet, daß** die Anode (3) der Elektrodialysevorrichtung von der anodenseitig ersten Zelleinheit durch eine kationenselektive Membran (12) getrennt ist.

7. Verfahren nach einem der Ansprüche 1 bis 6, **dadurch gekennzeichnet, daß** das Oniumsalz in einem protischen Lösemittel oder einem Gemisch aus zwei oder mehr protischen Lösemitteln gelöst ist.

8. Verfahren nach einem der Ansprüche 1 bis 7, **dadurch gekennzeichnet, daß** das Oniumsalz in einem Gemisch aus Wasser und einer wasserlöslichen, OH-Gruppen tragenden Verbindung oder einem Gemisch aus zwei oder mehr solcher Verbindungen gelöst ist.

9. Verfahren zur Reinigung von Oniumhydroxiden der Elemente N, S oder P durch Elektrodialyse in einer Elektrodialysevorrichtung mit einer Anode (3) einer Kathode (3) sowie einer oder mehreren Zelleinheiten, die jeweils einen Säuren (7) und einen Basenkreislauf aufweisen, bei dem eine in Wasser dissoziierbare Verunreinigungen enthaltende Lösung eines Oniumhydroxids der allgemeinen Formel I, worin M für N, S oder P; R¹, R², R³ und R⁴ jeweils unabhängig voneinander für einen linearen oder verzweigten, gesättigten oder ungesättigten aliphatischen, cycloaliphatischen, araliphatischen oder aromatischen Rest mit 1 bis 20 C-Atomen, X für OH und n für die Zahl 1 stehen, in den Basenkreislauf der Zelleinheit eingebracht und einer Elektrodialyse unterzogen wird, **dadurch gekennzeichnet, daß** jede Zelleinheit aus einer bipolaren Membran (1) und einer anionenselektiven Membran (4) besteht und sich anodenseitig zwischen der letzten anionenselektiven Membran und der Anode (3) eine bipolare Membran (8) oder eine kationenselektive Membran (12) befindet und die Kathode (2) der Elektrodialysevorrichtung von der kathodenseitig ersten Zelleinheit durch eine kationenselektive Membran (11) getrennt ist.

10. Verfahren nach Anspruch 9, **dadurch gekennzeichnet, daß** es wie in einem der Ansprüche 1 bis 8 definiert durchgeführt wird.

## Claims

1. A method of preparing onium hydroxides of the elements N, S or P by electrodialysis in an electrodialysis apparatus which includes an anode (3), a cathode (2) and one or more cell units each comprising one acid circuit (7) and one base circuit, wherein a solution (5) of an onium salt of the formula I 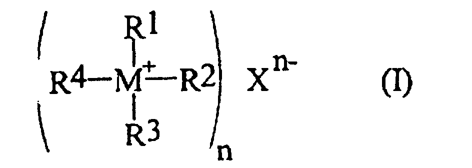 where M is N, S or P; R¹, R², R³ and R⁴ each, independently of one another, are a linear or branched, saturated or unsaturated aliphatic, cycloaliphatic, araliphatic or aromatic radical which is unsubstituted or which may be substituted by functional groups and has from 1 to 30 C atoms, or two of the radicals R¹ to R⁴ together with M form a heterocyclic ring, Xⁿ⁻ is an n-valent anion and n is a number from 1 to 4,
is introduced into the base circuit of the cell unit and subjected to an electrodialysis, **characterized in that** each cell unit comprises a bipolar membrane (1) and an anion-selective membrane (4), and a bipolar membrane (8) or a cation-selective membrane (12) is located on the anode side between the last anion-selective membrane and the anode (3), and the cathode (2) of the electrodialysis apparatus is separated from the first cell unit on the cathode side by a cation-selective membrane (11).

2. A method as claimed in claim 1, **characterized in that** R¹, R², R³ and R⁴ each, independently of one another, are a linear or branched aliphatic radical having from 1 to 4 C atoms.

3. A method as claimed in claim 1 or 2, **characterized in that** X is the anion of a Brönsted acid.

4. A method as claimed in any one of claims 1 to 3, **characterized in that** X is F, Cl, Br, I, SO_{4,} R⁵SO₄, HSO₄, CO₃, HCO₃, R⁵CO₃ or R⁵CO₂ or a mixture of two or more of these and R⁵ is a linear or branched, saturated or unsaturated aliphatic, cycloaliphatic, araliphatic or aromatic radical having from 1 to 30 C atoms.

5. A method as claimed in any one of claims 1 to 4, **characterized in that** M is N.

6. A method as claimed in any one of claims 1 to 5, **characterized in that** the anode (3) of the electrodialysis apparatus is separated from the first cell unit on the anode side by a cation-selective membrane (12).

7. A method as claimed in any one of claims 1 to 6, **characterized in that** the onium salt is dissolved in a protic solvent or a mixture of two or more protic solvents.

8. A method as claimed in any one of claims 1 to 7, **characterized in that** the onium salt is dissolved in a mixture of water and a water-soluble OH-carrying compound or a mixture of two or more such compounds.

9. A method of purifying onium hydroxides of the elements N, S or P by electrodialysis in an electrodialysis apparatus comprising an anode (3), a cathode (2) and one or more cell units which each include an acid circuit (7) and a base circuit wherein a solution of an onium hydroxide of the formula I containing impurities that can dissociate in water, in which formula (I) M is N, S or P; R¹, R², R³ and R⁴ each, independently of one another, are a linear or branched, saturated or unsaturated aliphatic, cycloaliphatic, araliphatic or aromatic radical having from 1 to 20 C atoms, X is OH and n is the number 1, is introduced into the base circuit of the cell unit and subjected to an electrodialysis, **characterized in that** each cell unit comprises a bipolar membrane (1) and an anion-selective membrane (4), and a bipolar membrane (8) or a cation-selective membrane (12) is located on the anode side between the last anion-selective membrane and the anode (3), and the cathode (2) of the electrodialysis apparatus is separated from the first cell unit on the cathode side by a cation-selective membrane (11).

10. A method as claimed in claim 9, **characterized in that** it is implemented as defined in any one of claims 1 to 8.

## Revendications

1. Procédé de fabrication d'hydroxydes d'onium des éléments N, S ou P par électrodialyse dans un dispositif d'électrodialyse qui présente une anode (3), une cathode (2) ainsi qu'une ou plusieurs unités cellulaires comportant respectivement un circuit d'acides (7) et un de bases, dans lequel une solution (5) d'un sel d'onium de la formule générale I 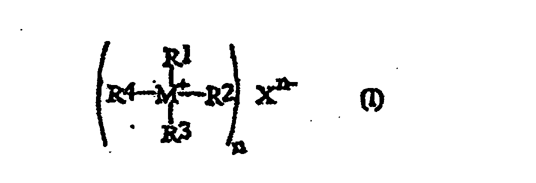 dans laquelle M signifie N, S ou P, R¹, R², R³ et R⁴, respectivement indépendants l'un de l'autre, signifient un reste aliphatique, cycloaliphatique, araliphatique ou aromatique, éventuellement substitué par des groupes fonctionnels, linéaire ou ramifié, saturé ou insaturé, ayant 1 à 30 atomes de C, ou deux des restes R¹ à R⁴ forment, avec M, un anneau hétérocyclique, X signifie un anion n-valent et n un nombre de 1 à 4,
est introduite dans le circuit des bases de l'unité cellulaire et soumise à une électrodialyse, **caractérisé en ce que** chaque unité cellulaire est constituée d'une membrane bipolaire (1) et d'une membrane sélective d'anions (4) et que, côté anode une membrane bipolaire (8) ou une membrane sélective de cations (12) se trouve entre la dernière membrane sélective d'anions et l'anode (3), et que la cathode (2) du dispositif d'électrodialyse est séparée, par une membrane sélective de cations (11), de la première unité cellulaire côté cathode.

2. Procédé selon la revendication 1, **caractérisé en ce que** R¹, R², R³ et R⁴, respectivement indépendants l'un de l'autre, signifient un reste aliphatique linéaire ou ramifié comportant 1 à 4 atomes de C.

3. Procédé selon la revendication 2, **caractérisé en ce que** X signifie l'anion d'un acide de Brönsted.

4. Procédé selon l'une des revendications 1 à 3, **caractérisé en ce que** X signifie F, Cl, Br, I, SO₄, R⁵SO₄, HSO₄, CO₃, HCO₃, R⁵CO₃, ou R⁵CO₂ ou un mélange de deux ou plusieurs de ceux-ci, et R⁵ signifie un reste aliphatique, cycloaliphatique, araliphatique ou aromatique linéaire ou ramifié, saturé ou insaturé, comportant 1 à 30 atomes de C.

5. Procédé selon l'une des revendications 1 à 4, **caractérisé en ce que** M signifie N.

6. Procédé selon l'une des revendications 1 à 5, **caractérisé en ce que** l'anode (3) du dispositif d'électrodialyse est séparée, par une membrane sélective de cations (12), de la première unité cellulaire côté cathode.

7. Procédé selon l'une des revendications 1 à 6, **caractérisé en ce que** le sel d'onium est dissous dans un solvant protique ou un mélange de deux ou plusieurs solvants protiques.

8. Procédé selon l'une des revendications 1 à 7, **caractérisé en ce que** le sel d'onium est dissous dans un mélange d'eau et d'une composition soluble dans l'eau, portant des groupes OH, ou un mélange de deux ou plusieurs de ces compositions.

9. Procédé d'épuration d'hydroxydes d'onium des éléments N, S ou P par électrodialyse dans un dispositif d'électrodialyse comportant une anode (3), une cathode (2) ainsi qu'une ou plusieurs unités cellulaires qui présentent respectivement un circuit d'acides (7) et un de bases, dans lequel une solution, contenant des impuretés dissociables dans l'eau, d'un hydroxyde d'onium de la formule générale I, dans laquelle M signifie N, S ou P, R¹, R², R³ et R⁴, respectivement indépendants l'un de l'autre, signifient un reste aliphatique, cycloaliphatique, araliphatique ou aromatique, linéaire ou ramifié, saturé ou insaturé, ayant 1 à 20 atomes de C, X signifie OH et n le nombre 1, est introduite dans le circuit des bases de l'unité cellulaire et soumise à une électrodialyse, **caractérisé en ce que** chaque unité cellulaire est constituée d'une membrane bipolaire (1) et d'une membrane sélective d'anions (4) et que, côté anode, une membrane bipolaire (8) ou une membrane sélective de cations (12) se trouve entre la dernière membrane sélective d'anions et l'anode (3), et que la cathode (2) du dispositif d'électrodialyse est séparée, par une membrane sélective de cations (11), de la première unité cellulaire côté cathode.

10. Procédé selon la revendication 9, **caractérisé en ce qu'**il est réalisé comme défini dans l'une des revendications 1 à 8.
